# EUROPÄISCHE PATENTANMELDUNG

(11) **EP 3 170 446 A1**
(43) Veröffentlichungstag der Anmeldung: **24.05.2017**
(21) Anmeldenummer: 15195694.3
(22) Anmeldetag: 20.11.2015
(51) Int. Cl.: A61B 5/00, A61B 5/0205, A61B 5/145, A61B 5/1455

(54) **VERFAHREN UND VORRICHTUNG ZUR NICHTINVASIVEN OPTISCHEN IN-VIVO-BESTIMMUNG DER GLUKOSEKONZENTRATION IN FLIESSENDEM BLUT**

(71) Anmelder: NIRLUS Engineering AG, 23560 Lübeck (DE)
(72) Erfinder: HERRMANN, Vera, 23560 Lübeck (DE)
(74) Vertreter: von dem Borne, Andreas

(57) **Zusammenfassung**

Es handelt sich um ein Verfahren und eine Vorrichtung zur nichtinvasiven optischen in-vivo-Bestimmung der Glukosekonzentration in fließendem Blut in einem Blutgefäß im Innern eines Körpers, wobei der Körper zur Markierung eines Blutgefäßes mit Ultraschallstrahlung mit einer Ultraschallfrequenz bestrahlt wird,
wobei der Körper mit dem Blutgefäß mit Licht mit zumindest einer ersten Lichtwellenlänge beleuchtet wird, bei der die Intensität des rückgestreuten Lichtes von der Glukosekonzentration abhängt,
wobei der Körper mit dem Blutgefäß mit Licht einer zweiten Lichtwellenlänge beleuchtet wird, die im Bereich einer Wasserabsorptionslinie liegt, deren Lage von der Temperatur des Blutes abhängt,
wobei das jeweils rückgestreute Licht mit zumindest einem Detektor erfasst wird,
wobei mit einer Auswerteeinheit aus den an dem Detektor gemessenen Detektorsignalen jeweils die mit einer von der Ultraschallfrequenz abhängigen Modulationsfrequenz modulierten Signalanteile extrahiert werden,
wobei aus dem bei der ersten Wellenlänge ermittelten Signalanteil ein Indikatorwert für die Glukosekonzentration ermittelt wird,
wobei der Indikatorwert zur Kompensation der Temperaturabhängigkeit mit dem Signalanteil der zweiten Lichtwellenlänge korrigiert wird.

## Beschreibung

Die Erfindung betrifft ein Verfahren zur nichtinvasiven optischen in-vivo-Bestimmung der Glukosekonzentration in fließendem Blut in einem Blutgefäß im Innern eines Körpers. Im Vordergrund der Erfindung steht dabei die optische Analyse mittels Licht, insbesondere mittels Laserstrahlung durch Auswertung des rückgestreuten Lichtes, wobei der Ort der Messung, nämlich die Blutbahn, mittels (gepulster) Ultraschallstrahlung "markiert" wird. Ziel ist im Rahmen der Erfindung die Bestimmung der Blutglukosekonzentration bzw. des Blutzuckerspiegels in-vivo, das heißt ohne direkte Kontaktierung des Blutes, so dass insbesondere auf eine Blutentnahme verzichtet werden kann. So besteht z. B. für Diabetiker das Bedürfnis eines schnellen und einfachen Messverfahrens, z B. mit einem kompakten und portablen Messgerät, das allenfalls durch Hautkontakt und ohne Verletzung der Haut schnell verlässliche Werte liefert.

Ein Verfahren zur optischen Messung von Eigenschaften von fließendem Blut mit Ultraschalllokalisierung ist z. B. aus der EP 1 601 285 B1 bekannt. Die Ultraschallstrahlung wird auf das Innere eines zentralen Blutgefäßes fokussiert und eine feste Pulslänge und Repititionszeit für die Ultraschallstrahlung wird vorgegeben. Außerdem werden eine Lichtquelle sowie eine benachbarte Detektionseinheit zum Erfassen des rückgestreuten Lichtes auf die Hautoberfläche über dem Blutgefäß derart positioniert, dass der Abstand zwischen Lichtquelle und der Mehrheit der Lichtrezeptoren der Detektionseinheit mit der Tiefe des untersuchtes Blutgewebes korrespondiert. Das Zielgewebe wird mit wenigstens zwei diskreten Lichtwellenlängen beleuchtet und das rückgestreute Licht wird gemessen und über die Detektorfläche und eine Vielzahl von Ultraschallimpulsen integriert. Das Ultraschallwellenfeld verursacht durch Wechselwirkung mit Blut und Gewebe Änderungen der optischen Eigenschaften, insbesondere des Reflexions- und Streuvermögens. Dies führt zu einer Modulation des rückgestreuten Lichtes mit der Frequenz der Ultraschallstrahlung, so dass sich im Zuge der Auswertung der modulierte Anteil extrahieren lässt.

Im Zusammenhang mit der Bestimmung der Blutglukosekonzentration wird in der DE 10 2006 036 920 B3 ein Verfahren zur spektrometrischen Bestimmung der Blutglukosekonzentration in pulsierend fließendem Blut beschrieben, wobei eine Wellenlänge aus dem Bereich 1560 bis 1630 nm, vorzugsweise 1600 nm eingestrahlt wird. Außerdem wird eine zweite Wellenlänge aus dem Wellenlängenbereich 790 bis 815 nm eingestrahlt und der Verhältnis der Transmissions- und/oder Streuvermögen dieser beiden Wellenlängen wird berechnet, wobei dieses Verhältnis in Relation zur Bluttemperatur als Indikatorwert zum Ablesen der Blutglukosekonzentration aus einer Kalibriertabelle dient. Die zweite Wellenlänge dient bei dem beschriebenen Verfahren der Kompensation des Einflusses der Glukosekonzentration an den Streuungskoeffizienten. Wesentlich für die Bestimmung der Glukosekonzentration auf diese Art ist eine zuverlässige Temperaturbestimmung. Außerhalb des menschlichen Körpers lässt sich dieses durch direkte Temperaturmessung realisieren. So lässt sich das in der DE 10 2006 036 920 B3 beschriebene Verfahren z. B. zum Monitoring des Blutzuckergehalts im Rahmen der Dialyse verwenden, da in diesem Fall die Möglichkeit besteht, die Temperatur des Blutes außerhalb des Körpers exakt zu bestimmen. Eine Umsetzung des in der DE 10 2006 036 920 B3 beschriebenen Verfahrens zur nichtinvasiven in-vivo-Bestimmung der Glukosekonzentration setzt ergänzend eine nichtinvasive Bestimmung der Temperatur des Blutes voraus.

Ein solches Verfahren zur nichtinvasiven, optischen Bestimmung der Temperatur eines Mediums innerhalb eines Körpers ist aus der DE 10 2008 006 245 A1 bekannt. Das zu untersuchende Medium wird mit infrarotem und/oder sichtbarem Licht im Bereich einer Absorptionslinie beleuchtet, deren Lage von der Temperatur des Mediums abhängt, wobei die Absorption des Lichtes im Bereich der Absorptionslinie gemessen und aus dieser Messung durch Vergleich mit Kalibrierungsdaten die Temperatur ermittelt wird. Wesentlich ist dabei, dass das Medium mit zumindest zwei diskreten Lichtwellenlängen beleuchtet wird, welche im Bereich der Absorptionslinie auf unterschiedlichen Seiten des Absorptionsmaximums liegen. Aus dem Verhältnis bzw. einem funktionellen Zusammenhang dieser beiden ermittelten Absorptionswerte zueinander wird zumindest ein von der Temperatur abhängiger Messwert oder eine von der Temperatur abhängige Messfunktion bestimmt, wobei aus diesem Messwert bzw. dieser Messfunktion durch Vergleich mit den zuvor aufgenommenen Kalibrierungsdaten die Temperatur bestimmt wird. Auch bei dieser optischen Temperaturmessung kann der Ort der Messung im Innern eines Körpers, z. B. einer Blutbahn, mittels gepulster Ultraschallstrahlung markiert werden. Im Vordergrund steht dabei die möglichst exakte Temperaturbestimmung, so dass insbesondere eine Temperaturkalibrierung erforderlich ist.

Die beschriebenen Verfahren ermöglichen bereits die in-vivo-Bestimmung der Blutglukosekonzentration im fließenden Blut im Innern eines Körpers. Die aus der EP 1 601 285 B1 beschriebene Ultraschalllokalisierung spielt dabei eine besondere Rolle. Durch Kombination mit dem aus der DE 10 2008 006 245 A1 beschriebenen Verfahren lässt sich auch die Temperatur des Blutes in-vivo ermitteln und bei der Auswertung berücksichtigen. Die insoweit bekannten Verfahren sind jedoch weiterentwicklungsfähig, um insbesondere die Qualität der Messung und auch die Handhabung einer entsprechenden Vorrichtung zu optimieren. - Hier setzt die Erfindung ein.

Der Erfindung liegt die Aufgabe zugrunde, ein Verfahren zu schaffen, welches eine vereinfachte und verbesserte nichtinvasive optische in-vivo-Bestimmung der Glukosekonzentration in fließendem Blut in einem Blutgefäß im Innern eines Körpers ermöglicht.

Zur Lösung dieser Aufgabe lehrt die Erfindung ein Verfahren zur nichtinvasiven optischen in-vivo-Bestimmung der Glukosekonzentration im fließenden Blut in einem Blutgefäß im Innern eines Körpers,
wobei der Körper zur Markierung eines Blutgefäßes mit (vorzugsweise gepulster) Ultraschallstrahlung mit einer Ultraschallfrequenz fus bestrahlt wird,
wobei der Körper mit dem Blutgefäß mit Licht mit zumindest einer ersten Lichtwellenlänge beleuchtet wird, bei der die Intensität des rückgestreuten Lichtes von der Glukosekonzentration abhängt,
wobei der Körper mit dem Blutgefäß zur Temperaturkompensation mit zumindest einer zweiten Lichtwellenlänge beleuchtet wird, die im Bereich einer Wasserabsorptionslinie liegt, deren Lage von der Temperatur des Blutes abhängt,
wobei das jeweils rückgestreute Licht mit zumindest einem Detektor erfasst wird,
wobei mit einer Auswerteeinheit aus den an dem Detektor gemessenen Detektorsignalen jeweils die mit einer von der Ultraschallfrequenz abhängigen Modulationsfrequenz modulierten Signalanteile extrahiert werden,
wobei aus dem bei der ersten Wellenlänge ermittelten Signalanteil ein Indikatorwert für die Glukosekonzentration ermittelt wird,
wobei der Indikatorwert zur Kompensation der Temperaturabhängigkeit mit dem Signalanteil der zweiten Lichtwellenlänge korrigiert wird.

Die Erfindung geht dabei zunächst einmal von der grundsätzlich bekannten Erkenntnis aus, dass sich Eigenschaften von fließendem Blut, wie z. B. die Glukosekonzentration, im Innern eines Körpers nichtinvasiv und in-vivo mit optischen Methoden messen lassen, wenn zugleich eine Markierung des Messortes mittels Ultraschallstrahlung erfolgt. Die Erfindung greift dabei auf bekannte Verfahren, z. B. EP 1 601 285 B1 oder DE 10 2008 006 245 A1 oder DE 10 2006 036 902 B3 zurück und entwickelt diese weiter. Im Vordergrund steht dabei die Bestimmung der Blutglukosekonzentration mit zumindest einer entsprechenden NIR-Wellenlänge aus einem Bereich von 600 nm bis 2500 nm. Aus diesem Bereich wird zumindest eine Wellenlänge ausgewählt, bei welcher die Intensität des rückgestreuten Lichtes von der Glukosekonzentration abhängt. Diese Abhängigkeit kann durch Wechselwirkung der Strahlung durch Streuung und/oder Absorption dominiert werden. So liegt es z. B. im Rahmen der Erfindung, eine erste Lichtwellenlänge im Bereich einer Glukoseabsorptionslinie bzw. Glukoseabsorptionsbande zu wählen, z. B. aus einem Bereich 1560 bis 1630 nm, vorzugsweise 1600 nm. Dabei kann z. B. auf die Erkenntnisse aus der DE 10 2006 036 920 B3 zurückgegriffen werden. Die Erfindung hat jedoch erkannt, dass zur Bestimmung der Glukosekonzentration nicht nur Wellenlängen aus dem Bereich von Absorptionsbanden der Glukose in Betracht kommen, sondern dass der Glukosegehalt auch die (dynamische) Lichtstreuung beeinflusst, z. B. durch den Brechzahlkontrast zwischen den Gewebebestandteilen, so dass auch Wellenlängen außerhalb von Glukoseabsorptionsbanden verwendet werden können. Im Übrigen ist es vorteilhaft, wenn die erste Wellenlänge aus einem Bereich ausgewählt wird, in dem die Intensität des rückgestreuten Lichtes nicht oder nicht wesentlich von der Oxygenisierung des Blutes abhängt. Insofern kann bevorzugt eine erste Wellenlänge aus einem Bereich von 790 bis 815 nm, vorzugsweise 800 bis 810 nm gewählt werden. Denn in diesem Bereich schneiden sich die beiden Absorptionskurven von Oxyhämoglobin und Desoxyhämoglobin, so dass die von der Glukosekonzentration abhängige Rückstreuung unabhängig vom Sauerstoffanteil des Blutes ist.

Alternativ kommen jedoch auch andere Wellenlängenbereiche in Betracht, in denen die Rückstreuung zwar von der Glukosekonzentration, jedoch nicht oder nur unwesentlich von der Sauerstoffbeladung des Blutes abhängt. So ist z. B. auch die Rückstreuung in dem bereits erwähnten Bereich um 1600 nm nahezu unabhängig von der Beladung mit Sauerstoff.

Folglich kann die erste Lichtwellenlänge im Rahmen einer ersten bevorzugten Alternative aus einem Bereich von 790 bis 815 nm, vorzugsweise 800 bis 810 nm ausgewählt werden.

In einer zweiten Alternative kann die erste Lichtwellenlänge aus einem Bereich von 1500 bis 1850 nm, z. B. 1550 bis 1800 nm, vorzugsweise 1550 bis 1750 nm ausgewählt werden.

Schließlich kommt für die erste Wellenlänge als dritte Alternative auch ein Bereich von 1000 bis 1400, z. B. 1100 bis 1400, vorzugsweise 1180 bis 1250 nm in Betracht.

Ergänzend erfolgt die Bestrahlung und Detektierung mit einer zweiten und vorzugsweise einer dritten Wellenlänge, die im Bereich einer Wasserabsorptionslinie liegen und mit denen eine Kompensation der Temperaturabhängigkeit realisiert werden kann. Darauf wird im Folgenden noch näher eingegangen.

Ergänzend zu der verwendeten ersten Wellenlänge, deren Rückstreuung von der Glukosekonzentration des Blutes abhängt, wird bevorzugt eine weitere (vierte) Lichtwellenlänge verwendet, die von der ersten Lichtwellenlänge abweicht, bei der die Intensität des rückgestreuten Lichtes jedoch ebenfalls von der Glukosekonzentration abhängt.

Wird z. B. die erste Wellenlänge aus dem Bereich 790 bis 815 nm verwendet, so kann als vierte Wellenlänge z. B. eine aus dem Bereich 1000 bis 1400, z. B. 1100 bis 1400, vorzugsweise 1180 bis 1250 nm verwendet werden. Alternativ kann eine vierte Wellenlänge aus dem Bereich 1500 bis 1850, z. B. 1050 bis 1800, vorzugsweise 1550 bis 1750 und besonders bevorzugt eine vierte Wellenlänge im Bereich einer Glukoseabsorptionslinie bzw. Glukoseabsorptionsbande verwendet werden.

Insgesamt kann bei der Auswahl der vierten Wellenlänge auf dieselben Bereiche zurückgegriffen werden, die bereits für die erste Wellenlänge genannt wurden, wobei für die erste Wellenlänge einerseits und die vierte Wellenlänge andererseits jedenfalls verschiedene Wellenlängen und besonders bevorzugt Wellenlängen aus verschiedenen der genannten Wellenlängenbereiche ausgewählt werden, um in der beschriebenen Weise die verschiedenen Einflüsse zu eliminieren.

Die Bezeichnung "erste" Wellenlänge einerseits und "vierte" Wellenlänge andererseits bezieht sich dabei auf den Fall, dass für die Temperaturkompensation mit zwei Wellenlängen (zweite Wellenlänge und dritte Wellenlänge) gearbeitet wird. Es versteht sich, dass in dem Fall, in dem für die Temperaturkompensation lediglich eine Wellenlänge (zweite Wellenlänge) verwendet wird, dann die beschriebene "vierte Wellenlänge" als "dritte Wellenlänge" verwendet wird. Im Übrigen liegt es im Rahmen der Erfindung, nicht nur die erste und gegebenenfalls vierte Wellenlänge für die Bestimmung der Glukosekonzentration, sondern gegebenenfalls auch weitere Wellenlängen aus den beschriebenen Bereichen (z. B. eine fünfte Wellenlänge) auszuwählen, um die Messung weiter zu optimieren.

Insgesamt besteht erfindungsgemäß die Möglichkeit, mehrere Wellenlängen aus unterschiedlichen Bereichen zu verwenden, bei denen die verschiedenen Einflussgrößen (z. B. Hämatokritwert, Oxygenisierung, Temperatur, Hämoglobin, glykiertes Hämoglobin ...) unterschiedlich groß sind, so dass durch geeignete Kombination von zumindest zwei Wellenlängen die durch die Glukose bestimmten Effekte möglichst gezielt extrahiert werden können. Dabei ist es zweckmäßig, dass bei Verwendung einer ersten Wellenlänge und einer vierten Wellenlänge und gegebenenfalls einer fünften Wellenlänge die ermittelten Signalanteile ins Verhältnis gesetzt werden können, um einen Indikatorwert für die Glukosekonzentration unabhängig von anderen Einflüssen zu ermitteln.

Die insoweit beschriebene Bestimmung der Blutglukosekonzentration mit Hilfe zumindest der ersten Wellenlänge und gegebenenfalls der vierten (oder auch fünften) Wellenlänge wird mit einer Ultraschallmarkierung kombiniert, die z. B. aus der EP 1 601 285 B1 bekannt ist. Von besonderer Bedeutung ist jedoch erfindungsgemäß die Tatsache, dass im Zuge der Bestimmung der Blutglukosekonzentration die Temperaturabhängigkeit ohne absolute Temperaturbestimmung und folglich auch ohne Temperaturkalibrierung unmittelbar im Zuge der Messung kompensiert wird. Erfindungsgemäß wird folglich auf die aus der DE 10 2008 006 245 A1 bekannten Erkenntnisse zur Temperaturbestimmung in fließendem Blut zurückgegriffen, ohne dass jedoch eine Temperaturkalibrierung und folglich eine absolute Temperaturbestimmung erfolgt. Vielmehr geht die Absorptionsmessung im Bereich der temperaturabhängigen Wasserabsorptionslinie unmittelbar in die Messung ein, so dass aus dem Zusammenspiel der Absorption / Streuung der Glukose und der temperaturabhängigen Wasserabsorption unmittelbar eine Kompensation der Temperaturabhängigkeit erfolgt.

Das erfindungsgemäße Verfahren zeichnet sich folglich durch die Kombination mehrerer Messungen mit zumindest zwei, vorzugsweise zumindest drei Lichtwellenlängen und folglich z. B. zwei bzw. drei Laserlichtquellen aus, wobei für sämtliche Wellenlängen eine Ultraschalllokalisierung erfolgt, so dass die aus der Blutbahn stammenden Signalanteile extrahiert werden können.

Für die Temperaturkompensation kann es ausreichen, lediglich die zweite Wellenlänge im Bereich einer Wasserabsorptionslinie einzustrahlen und auszuwerten. Besonders bevorzugt wird der Körper bzw. das Blutgefäß für die Kompensation der Temperatureinflüsse jedoch mit einer dritten Lichtwellenlänge beleuchtet, die im Bereich derselben Wasserabsorptionslinie liegt, wobei die zweite und die dritte Lichtwellenlänge auf unterschiedlichen Seiten des Absorptionsmaximums liegen und wobei die Kompensation der Temperaturabhängigkeit mit dem Verhältnis dieser beiden ermittelten Signalanteile zueinander vorgenommen wird. In diesem Fall wird das aus der DE 10 2008 006 245 A1 beschriebene Verfahren zur Temperaturbestimmung herangezogen, jedoch ohne entsprechende Temperaturkalibrierung, sondern zur einfachen Kompensation der Temperaturabhängigkeit unmittelbar im Zuge der Messung. In dieser bevorzugten Ausführungsform kommen folglich zumindest drei Lichtwellenlängen zum Einsatz, wobei die erste Lichtwellenlänge die Glukoseabhängigkeit betrifft, während die zweite Lichtwellenlänge und die dritte Lichtwellenlänge der Temperaturkompensation dienen.

Die zweite Lichtwellenlänge und/oder die dritte Lichtwellenlänge betragen vorzugsweise 600 bis 2500 nm, vorzugsweise 800 nm bis 1600 nm, z. B 950 nm bis 1000 nm. Versuche haben gezeigt, dass die Messung der Temperatur mit Hilfe von Infrarotlicht im Bereich der Wasserabsorptionsbande um 970 nm zu hervorragenden Ergebnissen führt. In diesem Fall wird dann zumindest eine Wellenlänge zwischen z. B. 950 und 970 nm und zumindest eine Wellenlänge zwischen z. B. 975 und 1000 nm eingesetzt. Es besteht aber auch die Möglichkeit, mit anderen Wasserabsorptionsbanden innerhalb des biologischen Fensters zu arbeiten, z. B. im Bereich der Wasserabsorptionsbande um 1450 nm. Grundsätzlich kommt jede Absorptionslinie in Betracht, deren Lage (Wellenlänge des Maximums) von der Temperatur abhängt. Sofern dabei gemäß DE 10 2008 006 245 A1 mit zwei Lichtwellenlängen im Bereich einer Wasserabsorptionslinie gearbeitet wird, wird das Medium - so wie in DE 10 2008 006 245 A1 beschrieben - mit zwei diskreten Lichtwellenlängen beleuchtet, welche im Bereich der Absorptionslinie auf unterschiedlichen Seiten des Absorptionsmaximums liegen, wobei aus dem Verhältnis bzw. einem funktionellen Zusammenhang dieser beiden ermittelten Absorptionswerte zueinander ein von der Temperatur abhängiger Korrekturwert bestimmt wird, der dann unmittelbar in die Temperaturkompensation einfließt. Diese Korrektur kann z. B. durch Bildung der Differenz der beiden beidseitig des Maximums liegenden Absorptionswerte oder durch Bestimmung der Steigung einer durch die Messpunkte verlaufenden Geraden bestimmt werden.

Im Vordergrund der Messung stehen der Einsatz mehrerer Lichtwellenlängen und deren Detektion. Dabei liegt es im Rahmen der Erfindung, die Messungen mit den einzelnen Wellenlängen zeitlich nacheinander durchzuführen, so dass dann ohne weiteres mit demselben Detektor gearbeitet werden kann. Alternativ liegt es jedoch auch im Rahmen der Erfindung, die Messung mit den verschiedenen Wellenlängen gleichzeitig durchzuführen. Bevorzugt wird dazu ebenfalls ein und derselbe Detektor eingesetzt. Um die Signale unterscheiden zu können, wäre es z. B. möglich, die einzelnen Laser mit unterschiedlichen Frequenzen zu modulieren, so dass dann durch Demodulierung des Detektorsignals die einzelnen Signale voneinander getrennt werden könnten. Alternativ besteht für eine gleichzeitige Aufnahme mehrerer Wellenlängen die Möglichkeit, mehrere Detektoren, z. B. drei oder vier Detektoren zu verwenden, die schmalbandig in entsprechenden Wellenlängenbereichen selektiv detektieren, z. B. auch mit der Anwendung von Spezialfiltern vor der Detektoroberfläche o.dgl..

Im Zusammenhang mit der Ultraschalllokalisierung kann insbesondere auf das aus der EP 1 601 285 B1 bekannte Verfahren zurückgegriffen werden, bei dem fokussierte Ultraschallstrahlung eingesetzt wird. Die Ultraschallstrahlung wird auf das Innere eines zentralen Blutgefäßes fokussiert und eine feste Pulslänge und Repititionszeit für die Ultraschallstrahlung wird vorgegeben. Dabei wird im Zuge der Auswertung der gesamte Lichtanteil, der mit der Frequenz der Ultraschallstrahlung moduliert ist, extrahiert, und zwar unabhängig davon, ob das Licht tatsächlich aus der Blutbahn oder eventuell aus angrenzendem Gewebe rückgestreut wurde. Dieses ist bei dieser Vorgehensweise deshalb möglich, weil die Ultraschallstrahlung auf die Blutbahn fokussiert wird, so dass der modulierte Anteil des Lichtes, der außerhalb der Blutbahn rückgestreut wird, gering ist.

Alternativ kann jedoch für die Ultraschalllokalisierung auch ein weiterentwickeltes Verfahren eingesetzt werden, bei dem auch mit nicht fokussierter Ultraschallstrahlung gearbeitet werden kann. Dabei wird der Körper zur Markierung eines Blutgefäßes mit Ultraschallstrahlung mit einer Ultraschallfrequenz fus bestrahlt und zugleich wird das Blutgefäß in der grundsätzlich bekannten Weise mit Licht mit der gewünschten Lichtwellenlänge beleuchtet und das rückgestreute Licht mit dem Detektor erfasst. Der außerhalb des Blutgefäßes aus dem Körper rückgestreute Lichtanteil ist mit einer Frequenz f_{MG} moduliert, welche der Frequenz fus der Ultraschallstrahlung entspricht. Demgegenüber ist der innerhalb des Blutgefäßes rückgestreute Lichtanteil aufgrund des Dopplereffektes in fließendem Blut mit einer um die Dopplerverschiebung f_{D} gegenüber der Frequenz fus der Ultraschallstrahlung verschobenen Frequenz f_{MB} moduliert. Mit der Auswerteeinheit wird dann aus dem an dem Detektor gemessenen Detektorsignal der mit der verschobenen Frequenz f_{MB} modulierte Signalanteil extrahiert. Bei dieser optimierten Variante ist gewährleistet, dass tatsächlich nur solche Lichtanteile des rückgestreuten Lichtes in die Auswertung einfließen, welche tatsächlich aus dem Blut rückgestreut werden. Bei diesem Aspekt geht die Erfindung von der Erkenntnis aus, dass die aus dem fließenden Blut einerseits und dem umgebenden Gewebe andererseits rückgestreuten Lichtanteile mit unterschiedlichen Modulationsfrequenzen moduliert sind. In dem umgebenden Gewebe ist die Modulationsfrequenz gleich der Ultraschallfrequenz. Im fließenden Blut erfolgt jedoch aufgrund des Dopplereffektes eine Modulation mit einer veränderten Frequenz. Erfindungsgemäß gelingt folglich unter Ausnutzung des Dopplereffektes die präzise Ortung der Blutbahn, und zwar unabhängig davon, ob mit fokussierter Ultraschallstrahlung gearbeitet wird oder nicht. Dabei ist es zweckmäßig, wenn der Körper mit gepulster Ultraschallstrahlung mit vorgegebener Pulslänge und Repititionszeit bestrahlt wird, wobei die Lichtintensität an dem Detektor in einem um eine Verzögerung zeitlich verschobenen Zeitfenster gemessen wird, welches der Pulslänge der Ultraschallstrahlung entspricht. Grundsätzlich erfolgt auch bei diesem Verfahren zunächst eine Lokalisierung des Blutgefäßes vor der optischen Messung durch akustische Analyse des aus dem Körper rückgestreuten Ultraschallechos.

Wie bereits erwähnt, zeichnet sich das erfindungsgemäße Verfahren mit Ultraschalllokalisierung dadurch aus, dass mit dem Detektor lediglich Intensitäten und folglich lediglich ein Photonenstrom gemessen wird. Dennoch werden im Zuge der Auswertung selbstverständlich durch geeignete Methoden die Signalanteile extrahiert, die mit der jeweils relevanten Frequenz in Abhängigkeit von der Ultraschallstrahlung moduliert sind. Dabei kann auf klassische Methoden zur Isolierung niedriger Frequenzen aus hochfrequenten Mischsignalen, z. B. auf eine Radar-Signal-Analyse zurückgegriffen werden.

Gegenstand der Erfindung ist auch eine Vorrichtung zur nichtinvasiven optischen in-vivo-Bestimmung der Glukosekonzentration in fließendem Blut in einem Blutgefäß im Innern eines Körpers nach einem Verfahren der beschriebenen Art, mit zumindest
- einer Ultraschallquelle,
- einer ersten Laserlichtquelle für die Erzeugung der ersten Lichtwellenlänge,
- einer zweiten Laserlichtquelle für die Erzeugung der zweiten Lichtwellenlänge,
- optional einer dritten Laserlichtquelle für die Erzeugung der dritten Lichtwellenlänge,
- einem optischen Detektor für die Detektion des rückgestreuten Lichtes,
- einer Steuer- und Auswerteeinheit, welche mit der Ultraschallquelle, den Laserlichtquellen und dem Detektor verbunden ist,
wobei mit der Steuer- und Auswerteeinheit aus den an dem Detektor gemessenen Detektorsignalen jeweils die mit einer von der Ultraschallfrequenz abhängigen Modulationsfrequenz modulierten Signalanteile extrahierbar sind,
wobei aus dem bei der ersten Wellenlänge ermittelten Signalanteil ein Indikatorwert für die Glukosekonzentration ermittelt wird und
wobei der Indikatorwert zur Kompensation der Temperaturabhängigkeit mit den Signalanteilen der zweiten Lichtwellenlänge korrigiert wird.

Bevorzugt weist die Vorrichtung zusätzlich eine dritte Laserlichtquelle für die Erzeugung der dritten Lichtwellenlänge auf, so dass für die Temperaturkompensation dann zwei Lichtwellenlängen im Bereich einer Wasserabsorptionslinie zur Verfügung stehen. Optional wird außerdem eine vierte Laserlichtquelle verwendet, die eine weitere "glukoseabhängige" Wellenlänge zur Verfügung stellt.

Bestandteil der Vorrichtung ist folglich eine Ultraschallquelle, die einen stark direktionalen Ultraschallstrahl erzeugt. Je nachdem, auf welches Verfahren zur Ultraschalllokalisierung zurückgegriffen wird, kann mit fokussierter oder nicht fokussierter Ultraschallstrahlung gearbeitet werden. Es wird ein homogener Ultraschalldruck in einer Größenordnung von etwa 1 MPa in der lateralen und axialen Richtung erzeugt. Besonders bevorzugt ist vorgesehen, dass der Einstrahlwinkel der Ultraschallstrahlung mit der Ultraschalleinheit veränderbar ist, und zwar vorzugsweise elektronisch, gegebenenfalls aber auch mechanisch. Die Einstellung des Winkels erleichtert die Suche des Blutgefäßes in unterschiedlichen Tiefen, um den Interaktionsort zwischen Licht und Ultraschall möglichst stets am gleichen Ort relativ zum Detektor halten zu können. Die verwendete Ultraschallfrequenz hängt davon ab, welche Art von Blutgefäß untersucht wird, wird z. B. im Bereich der Arteria radialis gemessen, kann eine Ultraschallfrequenz von z. B. 3,8 MHz zweckmäßig sein. Bevorzugt wird mit gepulster Ultraschallstrahlung gearbeitet, wobei die Pulswiederholungsrate von der Tiefe des untersuchten Ortes abhängt. Im Zuge der eigentlichen Messung wird Ultraschallstrahlung erzeugt, welche das optische Signal am optischen Detektor moduliert. Im Vorfeld der Messung ist es jedoch erforderlich, auch zur Lokalisierung der Blutbahn das Ultraschallecho aufzunehmen, so dass die Ultraschalleinheit einen Transducer aufweisen soll, der nicht nur die Erzeugung, sondern auch die Detektion der Ultraschallstrahlung ermöglicht.

Die Lichtquellen sind erfindungsgemäß bevorzugt als Laserlichtquellen ausgebildet, wobei mit zumindest drei, vorzugsweise aber mit vier Laserlichtquellen gearbeitet wird. Die Laserlichtquellen erzeugen kontinuierliches, monochromatisches und kohärentes Licht der jeweiligen Wellenlänge. Dabei liegt es im Rahmen der Erfindung, dass die Laser durch die Steuereinheit nach dem Messalgorithmus einzeln geschaltet werden, das heißt eingeschaltet werden oder ausgeschaltet werden. Dabei liegt es im Rahmen der Erfindung, die Messungen nacheinander durchzuführen, so dass dann eine einfache Auswertung in einem einzigen Detektor erfolgt. Alternativ können die Messungen auch zeitgleich erfolgen, wobei dann die einzelnen Laserstrahlungen mit jeweils unterschiedlichen Frequenzen moduliert werden, so dass das Detektorsignal entsprechend demoduliert werden kann, um dann auch bei gleichzeitiger Messung feststellen zu können, welcher Signalanteil welcher Lichteinstrahlung entspricht.

Um insgesamt ein kompaktes Messgerät für die Bestimmung der Blutglukosekonzentration zu schaffen, wird vorgeschlagen, sämtliche Laser an einem Träger in der Mitte eines Sensorkopfes anzuordnen, es ist aber eine Lasereinheit mit mehreren Lasern vorgesehen. Beim Einschalten der Laser nacheinander wäre es möglich, einen Treiber, ein Kühlsystem sowie ein Netzteil für alle Laser einzusetzen.

Erfindungsgemäß können unterschiedliche Detektoreinheiten zum Einsatz kommen. Die Detektoren selbst werden bevorzugt von Dioden gebildet. Es liegt z. B. im Rahmen der Erfindung, auf eine Detektoreinheit zurückzugreifen, bei der eine kreisringförmige Messfläche realisiert ist, wobei der Einstrahlpunkt des Laserlichtes im Zentrum dieser kreisringförmigen Messfläche angeordnet ist. Dabei kann z. B. auf eine aus der DE 10 2007 020 078 A1 bekannte Detektoranordnung zurückgegriffen werden. Hintergrund ist die Überlegung, dass die rückgestreuten Photonen umso weiter vom Einstrahlpunkt entfernt aus dem Gewebe austreten, je tiefer es im Gewebe gestreut wird. Bei Untersuchungen des Gewebes in einer ganz bestimmten Tiefe wird folglich statistisch betrachtet das Streulicht mit besonders hoher Intensität in einem bestimmten Abstand vom Einstrahlpunkt ermittelt, welcher in etwa der Hälfte der Tiefe des Streuzentrums entspricht. Diesen Umstand macht man sich zunutze und ordnet z. B. eine kreisringförmige Messfläche um den Einstrahlpunkt herum an, wobei der Durchmesser der kreisringförmigen Messfläche vorzugsweise in etwa der Tiefe des zu untersuchenden Bereichs entspricht. Der Umstand, dass die Intensität vom Abstand vom Einstrahlpunkt in Abhängigkeit von der Tiefe des Streuzentrums abhängt, kann aber auch bei anderem Detektordesign ausgenutzt werden.

Im Übrigen kann bei der Ausgestaltung des Detektors bei entsprechender Ortung mit Ultraschall berücksichtigt werden, dass die Intensitäten des Photonenstroms Zufallsvariable mit statistischen Eigenschaften sind. Wenn kohärentes Licht durch ein Medium "diffundiert", hat das emittierte Licht ein "gesprenkeltes Muster", man spricht von sogenannten "Speckles". Als Speckle bezeichnet man einen Lichtpunkt, in welchem das Signal kohärent ist. Die Durchschnittsfläche A eines typischen Speckles beträgt ca. A = α · λ², wobei λ die Wellenlänge des Lichtes ist und α im vorliegenden Fall zwischen 3 und 5 liegt. Die Detektion lässt sich optimieren, wenn eine möglichst geringe Anzahl von Speckles in einem beobachteten Moment den Detektor erreicht. Es wäre daher besonders vorteilhaft, eine Vielzahl kleiner Detektoren simultan auszulesen. Dieses ließe sich durch eine Mehrkanalelektronik mit entsprechendem Aufwand erreichen. Um den Aufbau des Detektors einfach und damit die Kosten gering zu halten, wird in bevorzugter Weiterbildung vorgeschlagen, den Detektor als Diodenarray oder Diodenzeile mit einer Vielzahl von Dioden auszubilden, die in einer Draufsicht quer bzw. senkrecht zur Richtung der Ultraschallstrahlung nebeneinander angeordnet sind. Die einzelnen Signale werden z. B. aufsummiert. Die Ultraschallstrahlung wird z. B. schräg unter einem vorgegebenen Einstrahlwinkel in dem Körper eingestrahlt und der Detektor wird - bezogen auf den Eintrittspunkt des Lichtes in den Körper - auf der gegenüberliegenden Seite angeordnet. Diese Ausgestaltung hat den Vorteil, dass die rückgestreuten Speckles von der Ultraschallstrahlung nicht mehrfach moduliert werden, so dass das Messergebnis verbessert wird. Alternativ zu einem Diodenarray oder einer Diodenzeile kann auch ein einzelner Detektor bzw. Einzeldetektor oder eine entsprechende Diode verwendet werden, der/die z. B. rechteckig ausgebildet sein kann und dessen lange Achse quer zur Ausbreitungsrichtung der Ultraschallstrahlung und/oder quer zum Lichtweg angeordnet sein kann.

Im Folgenden wird die Erfindung anhand einer lediglich ein Ausführungsbeispiel darstellenden Zeichnung näher erläutert. Es zeigen
- Fig. 1: eine erfindungsgemäße Vorrichtung in einer vereinfachten schematischen Darstellung,
- Fig. 2: die Anordnung der Ultraschallquelle und des Detektors in einer vereinfachten Ansicht und
- Fig. 3: die Situation gemäß Fig. 2 in einer anderen Darstellung.

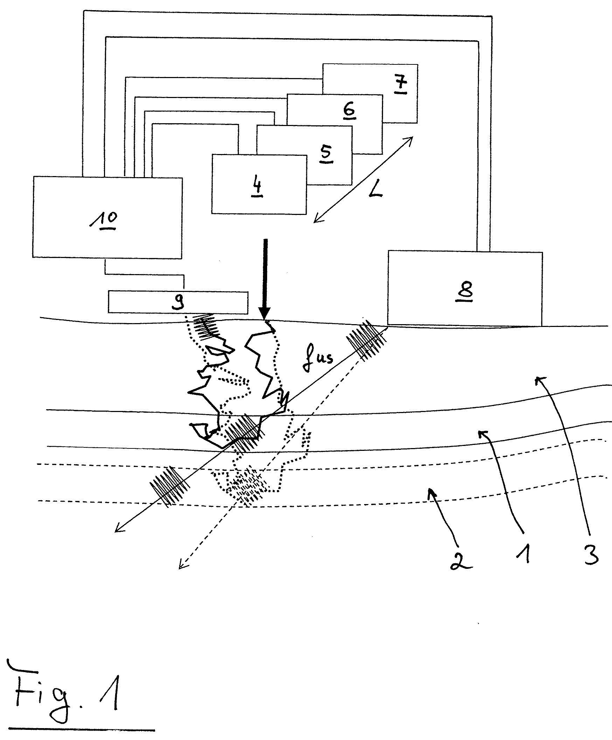

In Fig. 1 ist ein Körper mit zwei Blutgefäßen 1, 2 und dem an die Blutgefäße 1, 2 angrenzenden Gewebe 3 dargestellt. Zur nichtinvasiven optischen Messung der Glukosekonzentration innerhalb des fließenden Blutes sind eine Lasereinheit L mit mehreren Lasern 4, 5, 6, 7, eine Ultraschalleinheit 8, eine Detektoreinheit 9, eine Steuer- und Auswerteeinheit 10 vorgesehen. Der Körper mit dem Blutgefäß 1 wird mit Laserlicht aus den Lasern 4, 5, 6, 7 beleuchtet. Das rückgestreute Licht wird mit der Detektoreinheit 9 erfasst. Diese Detektoreinheit 9 misst lediglich Intensitäten, das heißt es erfolgt die Ermittlung des rückgestreuten Photonenstroms ohne Ortsauflösung oder (optische) Frequenzauflösung am Detektor.

Erfindungsgemäß wird der Körper zur Markierung des Blutgefäßes 1 mit Ultraschallstrahlung mit einer bestimmten Ultraschallfrequenz fus bestrahlt. Aufgrund der Wechselwirkung der Ultraschallstrahlung mit dem Blut bzw. Gewebe erfolgt eine Modulation der rückgestreuten Lichtanteile mit der Frequenz der Ultraschallstrahlung. Auf diese Weise kann die Auswerteeinheit 10 aus den an dem Detektor 9 gemessenen Detektorsignalen jeweils die mit einer von der Ultraschallfrequenz fus abhängigen Modulationsfrequenz modulierten Signalanteile extrahieren.

Erfindungsgemäß wird zur Bestimmung der Blutglukosekonzentration mit mehreren Lasern 4, 5, 6, 7 mit unterschiedlichen Lichtwellenlängen gearbeitet. Der erste Laser 4 weist eine Lichtwellenlänge auf, bei der der rückgestreute Signalanteil von der Glukosekonzentration abhängt und folglich die Glukosekonzentration repräsentiert und damit die Bestimmung eines entsprechenden Indikatorwertes ermöglicht. Dabei kann es sich z. B. um eine Lichtwellenlänge im Bereich zwischen 790 nm und 850 nm, z. B. um etwa 805 bis 808 nm handeln, denn in diesem Bereich kreuzen sich die beiden Absorptionskurven von Oxyhämoglobin und Desoxyhämoglobin, so dass bei dieser Wellenlänge die Absorption und damit auch der Anteil des rückgestreuten Lichtes unabhängig vom Beladungszustand mit Sauerstoff ist.

Ergänzend kann ein weiterer Laser, der in diesem Fall als vierter Laser 5 bezeichnet wird, verwendet werden, um eine weitere Lichtwellenlänge einzustreuen, bei der die Rückstreuung von der Glukosekonzentration abhängt. Dieser vierte Laser kann z. B. eine Wellenlänge aus einem Bereich von 1180 nm bis 1250 nm oder alternativ auch eine Wellenlänge aus einem Bereich von 1550 nm bis 1750 nm aufweisen.

Von besonderer Bedeutung ist die Tatsache, dass die weiteren Laser 6, 7, nämlich der zweite Laser 6 und gegebenenfalls der dritte Laser 7 für die Temperaturkompensation eingesetzt werden. Die zweite Lichtwellenlänge kann z. B. 950 bis 970 nm betragen und die dritte Lichtwellenlänge kann z. B. 975 nm bis 1000 nm betragen, so dass diese beiden Lichtwellenlängen auf unterschiedlichen Seiten des Absorptionsmaximums einer Wasserabsorptionslinie bei z. B. 970 nm liegen. Das Verhältnis der beiden Signalanteile, die auf die Rückstreuung der zweiten Lichtwellenlänge und der dritten Lichtwellenlänge zurückgehen, hängt sehr empfindlich von der Temperatur des Mediums ab, so dass diese Werte dann unmittelbar für eine Kompensation der Temperaturabhängigkeit eingesetzt werden können. Wichtig ist dabei die Tatsache, dass keine absolute Temperaturmessung erforderlich ist und folglich auch keine vorherige Temperaturkalibrierung vorgesehen ist. Es ist vollkommen ausreichend, im Zuge der Bestimmung der Glukosekonzentration unter Ausnutzung der ersten Wellenlänge (und gegebenenfalls der vierten Wellenlänge) die zweite und dritte Wellenlänge ebenfalls zu registrieren und die Messwerte entsprechend zu korrigieren.

In Fig. 1 ist im Übrigen erkennbar, dass dort zwei Blutgefäße 1, 2 untereinander liegen. Dennoch gelingt im Rahmen der Erfindung eine einwandfreie Lokalisierung und Trennung der Signale. Dieses hängt zunächst einmal damit zusammen, dass das Streulicht - so wie z. B. in der DE 10 2007 020 078 A1 beschrieben - statistisch betrachtet mit besonders hoher Intensität in einem bestimmten Abstand vom Einstrahlpunkt (des Lichtes) auftritt, und zwar in einem Abstand vom Einstrahlpunkt, welcher in etwa der Hälfte der Tiefe des Streuzentrums entspricht. Auf diese Weise ist bei einer bestimmten Geometrie das eventuell aus einer anderen Lage rückgestreute Signal an dem entsprechend angeordneten Detektor wesentlich geringer als das relevante Signal. In Fig. 1 erkennt man das beispielhaft an den beiden dargestellten Situationen. Ausgewertet werden soll das oben liegende Blutgefäß 1. Wird mit Hilfe einer variablen Winkeleinstellung der Ultraschallquelle 8 der Einstrahlwinkel der Ultraschallstrahlung variiert, so können auch rückgestreute Photonen auf den Detektor 9 treffen, die im Bereich der unteren Blutbahn 2 moduliert sind. Diese gestrichelt angedeutete Situation führt dann jedoch zu deutlich geringeren Intensitäten. Das Verfahren wird dadurch optimiert, dass mit gepulster Ultraschallstrahlung gearbeitet wird, wobei die Ultraschallstrahlung eine vorgegebene Pulslänge und Repititionszeit aufweist und wobei die Lichtintensität an dem Detektor 9 dann in einem um eine Verzögerung zeitlich verschobenen Zeitfenster gemessen wird, welches der Pulslänge der Ultraschallstrahlung entspricht. Auf diese Weise kann ebenfalls sichergestellt werden, dass dann tatsächlich nur die aus dem Bereich der oberen Blutbahn rückgestreuten Photonen ermittelt werden. Dabei ist wichtig, dass der Detektor in Fig. 1 deutlich überdimensional dargestellt ist. Für eine Selektion zwischen den Signalen untereinanderliegender Blutbahnen ist es vorteilhaft, wenn mit einem lediglich sehr kleinen Detektor gearbeitet wird, da dann aufgrund der beschriebenen statistischen Abhängigkeit sichergestellt ist, dass das Licht aus einer bestimmten Tiefe mit besonders hoher Intensität rückgestreut wird. Bevorzugt sollte der Detektor eine Größe in der Größenordnung eines Speckles aufweisen. Die Durchschnittsfläche eines Speckles beträgt ca. A = α · λ², wobei λ die Wellenlänge des Lichtes ist und α in diesem Fall zwischen 3 und 5 liegt.

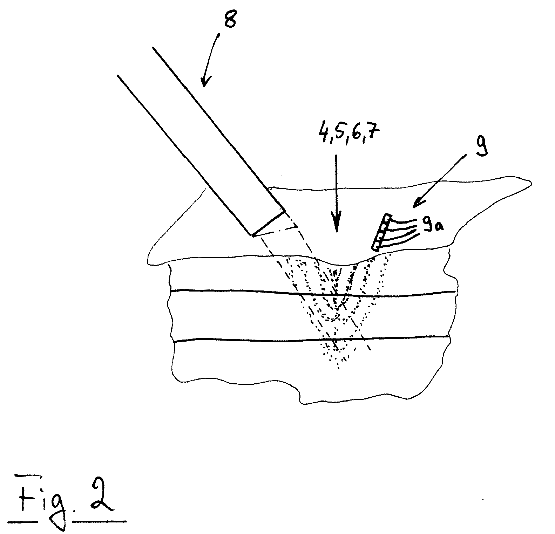

In Fig. 2 ist eine bevorzugte Anordnung der einzelnen Komponenten im Zuge der Messung schematisch angedeutet. Es ist erkennbar, dass die Ultraschallstrahlung bevorzugt unter einem vorgegebenen Winkel schräg in den Körper eingestrahlt wird und dass der Detektor 9 relativ zum eingestrahlten Licht der gegenüberliegenden Seite angeordnet wird. In dem dort dargestellten Ausführungsbeispiel ist als Detektor 9 ein Diodenarray mit mehreren einzelnen Dioden 9a vorgesehen, wobei das Signal aufsummiert wird. Durch die dargestellte Situation wird vermieden, dass rückgestreute Photonen durch die Ultraschallstrahlung auf dem Rückweg durch das Gewebe zusätzlich mehrfach moduliert werden. Damit lässt sich das Signal/Rauschverhältnis verbessern.

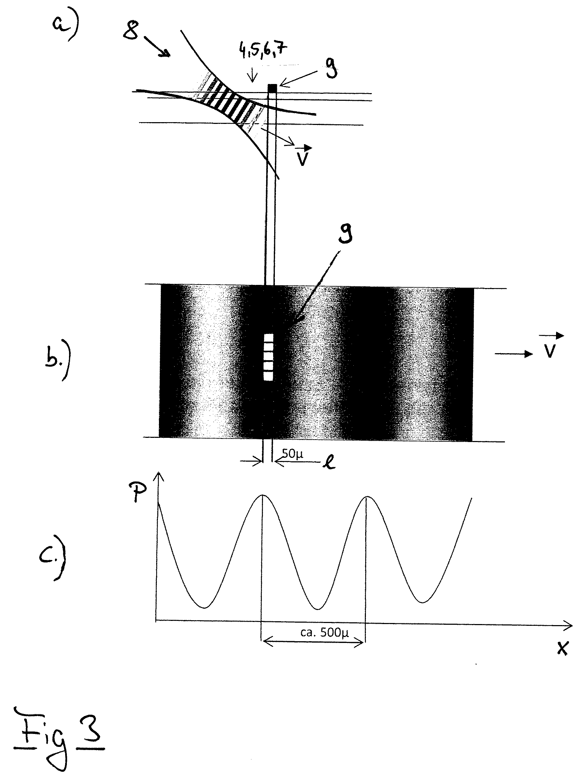

Die Situation wird auch anhand von Fig. 3 verdeutlicht. Dort ist im oberen Bereich a) die Situation gemäß Fig. 2 in einer Seitenansicht dargestellt und im mittleren Bereich b) in einer Draufsicht. Im mittleren Bereich b) ist grafisch die Variation des Ultraschalldruckes angedeutet. Im unteren Bereich c) von Fig. 3 ist dementsprechend der Ultraschalldruck P in Abhängigkeit vom Ort x gezeigt. Dabei ist in Fig. 3b) und 3c) der Ultraschalldruck P in Abhängigkeit vom Weg bzw. der Länge X in unterschiedlichen Darstellungen gezeigt. Der Geschwindigkeitsvektor V der Ultraschallwelle ist angedeutet. Es ist erkennbar, dass der Detektor 9 in Richtung der Ausbreitung der Ultraschallwelle eine sehr geringe Länge I aufweist, die im Ausführungsbeispiel 50 µm beträgt. Sie kann z. B. zwischen 10 µm und 100 µm liegen. Durch diese Dimensionierung soll zunächst einmal erreicht werden, dass eine möglichst geringe Anzahl von Speckles in einem beobachten Moment den Detektor 9 erreichen. Da das Signal durch eine solche geringe Dimensionierung und folglich eine geringe Länge I gering wird, sind mehrere Detektorelemente 9a nebeneinander angeordnet, so dass sich das Signal damit erhöhen lässt. Die geringe Länge I des Detektors hat im Übrigen den Vorteil, dass aufgrund der beschriebenen statistischen Effekte eine bessere Trennung zwischen Signalen möglich wird, die eventuell aus übereinander liegenden Blutbahnen resultieren können. Eine geringe Ausdehnung des Detektors ist folglich vorteilhaft. Gemeint ist dabei eine geringe Ausdehnung des Detektors in der Richtung, die durch den Abstand zwischen dem Einstrahlpunkt der Laserstrahlung und dem Detektor definiert wird. Diese Richtung kann auch der Richtung der Ausbreitung der Ultraschallwelle entsprechen. Dazu wird auf die Figuren verwiesen. Alternativ zu einem Detektor mit einzelnen Detektorelementen kann auch ein (z. B. rechteckiger) Einzeldetektor verwendet werden, der ebenso mit geringer Länge und größerer Breite dimensioniert und angeordnet werden kann.

Auch wenn - wie beschrieben - erfindungsgemäß keine Temperaturkalibrierung erforderlich ist, da ohne absolute Temperaturbestimmung lediglich eine temperaturabhängige Kompensation erfolgt, ist zur Bestimmung der Glukosekonzentration zuvor eine Kalibrierung des Systems erforderlich. Dazu können z. B. mit dem System in-vivo an geeigneten Probanden diverse Glukosesituationen mit einem Goldstandardreferenzsystem durchgeführt werden. Da durch die optische Messung auch die Information über den Hämatokritwert geliefert werden kann, ist es auch möglich, die korrekte Messung vollblut- oder plasmakalibriert zu erhalten.

Beim eigentlichen Messvorgang kann dann ohne weitere Kalibrierung gearbeitet werden. Der Detektor kann z. B. auf den Körper, z. B. auf den Unterarm gesetzt werden, direkt über einem Blutgefäß, z. B. der Arteria radialis. Dabei ist zu beachten, dass ein Ort gewählt werden sollte, bei dem das Blutgefäß nicht zu tief liegt, wobei eine ideale Tiefe weniger als 1 cm beträgt. Zunächst wird mit den bekannten Maßnahmen über das Ultraschallsystem die Arterie gesucht. Dieses kann durch Auswertung der rückgestrahlten Ultraschallstrahlung akustisch durchgeführt werden. Nach dem Auffinden wird die optische Messung automatisch gestartet. Das optische Messen besteht aus einer optimierten Folge von Lichtimpulsen der jeweiligen Wellenlängen, um alle physiologisch veränderten Streuungs- und Absorptionssituationen während mehrerer Herzpulse abzutasten. Die Signale werden analog/digital ausgewertet und in Rohdatentabellen/Arrays gespeichert. Anschließend erfolgt die Auswertung der auf diese Weise ermittelten Indikatorwerte und ein Vergleich mit entsprechenden Kalibrierungsdaten zur Bestimmung der Blutglukosekonzentration.

## Patentansprüche

1. Verfahren zur nichtinvasiven optischen in-vivo-Bestimmung der Glukosekonzentration in fließendem Blut in einem Blutgefäß im Innern eines Körpers, wobei der Körper zur Markierung eines Blutgefäßes mit Ultraschallstrahlung mit einer Ultraschallfrequenz bestrahlt wird,
wobei der Körper mit dem Blutgefäß mit Licht mit zumindest einer ersten Lichtwellenlänge beleuchtet wird, bei der die Intensität des rückgestreuten Lichtes von der Glukosekonzentration abhängt,
wobei der Körper mit dem Blutgefäß mit Licht einer zweiten Lichtwellenlänge beleuchtet wird, die im Bereich einer Wasserabsorptionslinie liegt, deren Lage von der Temperatur des Blutes abhängt,
wobei das jeweils rückgestreute Licht mit zumindest einem Detektor erfasst wird,
wobei mit einer Auswerteeinheit aus den an dem Detektor gemessenen Detektorsignalen jeweils die mit einer von der Ultraschallfrequenz abhängigen Modulationsfrequenz modulierten Signalanteile extrahiert werden,
wobei aus dem bei der ersten Wellenlänge ermittelten Signalanteil ein Indikatorwert für die Glukosekonzentration ermittelt wird,
wobei der Indikatorwert zur Kompensation der Temperaturabhängigkeit mit dem Signalanteil der zweiten Lichtwellenlänge korrigiert wird.

2. Verfahren nach Anspruch 1, wobei der Körper bzw. das Blutgefäß für die Kompensation der Temperatureinflüsse mit einer dritten Lichtwellenlänge beleuchtet wird, die im Bereich derselben Wasserabsorptionslinie liegt, wobei die zweite und dritte Lichtwellenlänge auf unterschiedlichen Seiten des Absorptionsmaximums liegen und wobei die Kompensation der Temperaturabhängigkeit mit dem Verhältnis dieser beiden ermittelten Signalanteile zueinander vorgenommen wird.

3. Verfahren nach Anspruch 1 oder 2, wobei der Körper mit dem Blutgefäß mit einer von der ersten Lichtwellenlänge verschiedenen vierten Lichtwellenlänge beleuchtet wird, bei der die Intensität des rückgestreuten Lichtes ebenfalls von der Glukosekonzentration abhängt.

4. Verfahren nach einem der Ansprüche 1 bis 3, wobei Lichtwellenlängen in einem Bereich von 600 nm bis 2500 nm verwendet werden.

5. Verfahren nach einem der Ansprüche 1 bis 4, wobei die erste und/oder die vierte Lichtwellenlänge aus einem Bereich ausgewählt sind, in dem die Intensität des rückgestreuten Lichtes nicht oder nicht wesentlich von der Oxygenisierung des Blutes abhängt.

6. Verfahren nach einem der Ansprüche 1 bis 5, wobei die erste oder die vierte Lichtwellenlänge aus einem Bereich von 790 nm bis 815 nm, vorzugsweise 800 nm bis 810 nm ausgewählt ist.

7. Verfahren nach einem der Ansprüche 1 bis 6, wobei die erste Lichtwellenlänge oder die vierte Lichtwellenlänge aus einem Bereich von 1000 nm bis 1400 nm, z. B. 1100 nm bis 1400, vorzugsweise 1180 nm bis 1250 nm ausgewählt ist.

8. Verfahren nach einem der Ansprüche 1 bis 7, wobei die erste Lichtwellenlänge oder die vierte Lichtwellenlänge aus einem Bereich von 1500 nm bis 1850 nm, z. B. 1550 nm bis 1800 nm, vorzugsweise 1550 nm bis 1750 nm ausgewählt ist.

9. Verfahren nach einem der Ansprüche 1 bis 8, wobei die zweite Lichtwellenlänge und/oder die dritte Lichtwellenlänge 600 nm bis 2500 nm, vorzugsweise 800 nm bis 1650 nm, z. B. 950 bis 1000 nm beträgt.

10. Verfahren nach Anspruch 9, wobei die zweite Lichtwellenlänge 950 bis 970 nm und die dritte Lichtwellenlänge 975 bis 1000 nm beträgt.

11. Verfahren nach einem der Ansprüche 1 bis 10, wobei die Messungen mit den verschiedenen Wellenlängen zeitlich nacheinander durchgeführt werden.

12. Verfahren nach einem der Ansprüche 1 bis 10, wobei die Messungen mit den verschiedenen Wellenlängen gleichzeitig durchgeführt werden.

13. Verfahren nach einem der Ansprüche 1 bis 12, wobei mit der Auswerteeinheit aus den jeweiligen Detektorsignalen die mit der Ultraschallfrequenz modulierten Lichtanteile extrahiert werden, wobei die Ultraschallstrahlung bevorzugt auf die Blutbahn fokussiert wird.

14. Verfahren nach einem der Ansprüche 1 bis 13, wobei die jeweils außerhalb des Blutgefäßes aus dem Körper rückgestreuten Lichtanteile mit einer Frequenz moduliert sind, welche der Frequenz der Ultraschallstrahlung entspricht,
wobei die jeweils innerhalb des Blutgefäßes rückgestreuten Lichtanteile aufgrund des Dopplereffektes in fließendem Blut mit einer um die Dopplerverschiebung gegenüber der Frequenz der Ultraschallstrahlung verschobenen Frequenz moduliert sind und
wobei mit der Auswerteeinheit aus den jeweils an dem Detektor gemessenen Detektorsignalen die mit der verschobenen Frequenz modulierten Signalanteile extrahiert werden.

15. Vorrichtung zur nichtinvasiven optischen in-vivo-Bestimmung der Glukosekonzentration in fließendem Blut in einem Blutgefäß im Innern eines Körpers nach einem Verfahren nach einem der Ansprüche 1 bis 14, mit zumindest
- einer Ultraschallquelle (8),
- einer ersten Laserlichtquelle (4) für die Erzeugung der ersten Lichtwellenlänge,
- einer zweiten Laserlichtquelle (5) für die Erzeugung der zweiten Lichtwellenlänge,
- einem optischen Detektor (9) für die Detektion des rückgestreuten Lichtes,
- einer Steuer- und Auswerteeinheit (10), welche mit der Ultraschallquelle (8), den Laserlichtquellen (4, 5, 6) und dem Detektor (9) verbunden ist,
wobei mit der Auswerteeinheit (10) aus den an dem Detektor (9) gemessenen Detektorsignalen jeweils die mit einer von der Ultraschallfrequenz (fus) abhängigen Modulationsfrequenz modulierten Signalanteile extrahierbar sind,
wobei aus dem bei der ersten (4) Wellenlänge ermittelten Signalanteile ein Indikatorwert für die Glukosekonzentration ermittelt wird und
wobei der Indikatorwert zur Kompensation der Temperaturabhängigkeit mit dem Signalanteil der zweiten Lichtwellenlänge (6) korrigiert wird.

16. Vorrichtung nach Anspruch 15, mit einer dritten und gegebenenfalls vierten Laserlichtquelle (7) für die Erzeugung der dritten bzw. vierten Lichtwellenlänge.

17. Vorrichtung nach Anspruch 15 oder 16, **dadurch gekennzeichnet, dass** der Einstrahlwinkel der Ultraschallstrahlung mit der Ultraschalleinheit (8) veränderbar ist, z. B. elektronisch oder/oder mechanisch.

18. Vorrichtung nach einem der Ansprüche 15 bis 17, **dadurch gekennzeichnet, dass** der Detektor (9) als Diodenarray mit einer Vielzahl von Dioden (9a) ausgebildet ist, die in einer Draufsicht auf den Körper quer zur Ausbreitungsrichtung der Ultraschallstrahlung und/oder quer zum Lichtweg nebeneinander angeordnet sind oder dass der Detektor als z. B. rechteckiger Einzeldetektor ausgebildet ist, der mit seiner Längsachse quer zur Ausbreitungsrichtung der Ultraschallstrahlung und/oder quer zum Lichtweg angeordnet ist.
